Europäisches Patentamt

European Patent Office

Office européen des brevets

(11) Veröffentlichungsnummer: **0 146 699**
**A1**

## (12) EUROPÄISCHE PATENTANMELDUNG

(21) Anmeldenummer: **84111617.1**

(22) Anmeldetag: **28.09.84**

(51) Int. Cl.⁴: **A 61 B 19/00**
**A 61 B 6/12**

(30) Priorität: **22.12.83 CH 6845/83**

(43) Veröffentlichungstag der Anmeldung:
**03.07.85 Patentblatt 85/27**

(84) Benannte Vertragsstaaten:
**AT DE FR GB IT**

(71) Anmelder: **GEBRÜDER SULZER AKTIENGESELLSCHAFT**
**Zürcherstrasse 9**
**CH-8401 Winterthur(CH)**

(72) Erfinder: **Bähler, André**
**Kreuzstrasse 46**
**CH-8008 Zürich(CH)**

(72) Erfinder: **Gschwend, Norbert, Prof. Dr.-med.**
**Neumünsterallee 3**
**CH-8008 Zürich(CH)**

(72) Erfinder: **Munziger, Urs, Dr.-med.**
**Feldstrasse 18**
**CH-8703 Erlenbach(CH)**

(72) Erfinder: **Scheier, Heinrich, Prof. Dr.-med.**
**Neumünsterallee 3**
**CH-8008 Zürich(CH)**

(74) Vertreter: **Dipl.-Ing. H. Marsch Dipl.-Ing. K. Sparing**
**Dipl.-Phys.Dr. W.H. Röhl Patentanwälte**
**Rethelstrasse 123**
**D-4000 Düsseldorf(DE)**

(54) **Markierungsimplantat.**

(57) Die aus Kontrastkörper (1) und Verankerungskörper (2) bestehenden Implantate werden mit Hilfe einer Struktur des Verankerungskörpers (2) im Knochen ortsfest fixiert und bilden so im Knochen unverrückbare Bezugspunkte für die Ausmessung von Röntgenbildern.

Fig. 1

EP 0 146 699 A1

0146699

P.5854/Wg/IS

Gebrüder Sulzer, Aktiengesellschaft, Winterthur/Schweiz

## Markierungsimplantat

Die Erfindung betrifft ein Markierungsimplantat zur Festlegung eines Bezugspunktes im menschlichen Knochengewebe.

Zu verschiedenen Zeiten - beispielsweise in Abständen eines Jahres oder länger - aufgenommene Röntgenbilder implantierter Gelenkendoprothesen dienen dazu, Verschiebungen, beispielsweise ein Einsinken oder "Setzen" der Implantate im Knochen oder Verschiebungen des Implantats infolge Knochenabbau möglichst frühzeitig festzustellen. Bei zu verschiedenen Zeiten gemachten Aufnahmen können dabei durch unterschiedliche Abbildungsmassstäbe bei den einzelnen Röntgenbildern und/oder durch unterschiedliche Lagen der durch das ersetzte Gelenk verbundenen Glieder relativ zur Aufnahmerichtung bei den verschiedenen Aufnahmen nicht vorhandene Aenderungen im Sitz der Prothese vorgetäuscht werden. Es ist daher notwendig, dass auf jeder Aufnahme im Knochen ortsfeste Bezugspunkte vorhanden sind, aus deren Abständen und Lagen zueinander Aenderungen des Abbildungsmassstabes und der Lage der Knochen relativ zur Aufnahmerichtung ermittelt werden. Bestimmt man - unter Berücksichtigung der genannten Lage- und Massstabsänderungen - die geometrischen Beziehungen dieser Bezugspunkte zu markanten Punkten der Prothese, so ist es möglich, die erwähnten "Verschiebungen" der Prothese festzustellen und unter Umständen quantitativ zu erfassen.

Aufgabe der Erfindung ist es, ein Implantat zu schaffen, mit dessen Hilfe ein ortsfester Bezugspunkt im Knochen "erzeugt" werden kann. Als Lösung dieser Aufgabe schlägt die Erfindung einen im Röntgenbild zu seiner Umgebung in Kontrast stehenden Kontrastkörper vor, dessen Raumform so ausgebildet ist, dass seine Projektion auf die Filmebene für jede mögliche Einstrahlrichtung eine geometrische Bestimmung desselben Raumpunktes ermöglicht und einen röntgenographisch durchlässigen Verankerungskörper,durch den der Kontrastkörper im Knochen ortsfest gehalten ist.

Die Abmessungen des Kontrastkörpers, der beispielsweise aus einem starke Röntgenkontraste erzeugenden Metall – also einem Metall möglichst grosser Dichte – besteht, werden so klein wie möglich gehalten, so dass der Kontrastkörper als Ganzes als Bezugspunkt dienen kann. Seine Dimensionen werden nach unten zum einen durch die Forderung begrenzt, dass durch sie auf dem Röntgenbild noch ein einwandfrei identifizierbarer Kontrastpunkt erzeugt werden muss; zum anderen soll selbstverständlich der Aufwand für seine Herstellung nicht zu gross werden.

Als vorteilhafteste geometrische Form des Kontrastkörpers hat sich eine Kugel erwiesen, deren Durchmesser mindestens 0,2 mm betragen sollte; es ist jedoch auch möglich, ihn beispielsweise als Würfel oder als Rundstab oder Zylinder mit abgerundeten oder kegelförmig zulaufenden Enden auszubilden. Sind die Abmessungen des Kontrastkörpers so gross, dass sein Bild nicht mehr als "Punkt" angenommen werden kann, so ist es weiterhin möglich, in seinem Bild einen Punkt, beispielsweise den Mittelpunkt bei einer Kugel grösseren Durchmessers,zu bestimmen.

Der Verankerungskörper besteht im einfachsten Falle aus
einem, in der Implantat-Technik üblichen Kunststoff, z.B.
Polyäthylen; er weist mit Vorteil eine zur ortsfesten
Fixierung im Knochen geeignete Struktur auf, die z.B.
in Rippen, widerhakenartigen Verzahnungen, Lamellen oder
einem Gewinde bestehen kann. Weiterhin ist es zweckmässig,
wenn der Verankerungskörper Mittel für den Eingriff eines
Setz- und/oder Ausziehinstrumentes aufweist.

Ist der Kontrastkörper gegen Körperflüssigkeit nicht
korrosionsbeständig, so ist es schliesslich vorteilhaft,
wenn er in ein, als Verankerungskörper dienendes, flüssigkeitsdichtes Gehäuse eingelegt ist.

Im Folgenden wird die  Erfindung anhand von Ausführungsbeispielen im Zusammenhang mit der Zeichnung näher erläutert.

Fig. 1 ist ein Längsschnitt I-I von Fig. 2 durch
eine erste Ausführungsform des erfindungsgemässen Implantats;

Fig. 2 ist eine Ansicht von Fig. 1 von links;

Fig. 3 ist, teilweise im Schnitt, ein zweites
Ausführungsbeispiel, in dem

Fig. 4 wiederum eine Ansicht von links darstellt;

Fig. 5 und 6 zeigen in einer Aufsicht auf eine
Sagittal- und eine Frontalebene den Einsatz
der erfindungsgemässen Implantate in Verbindung
mit einer Kniegelenkprothese, wobei die Darstellung
dem Erscheinungsbild einer Röntgenaufnahme entspricht.

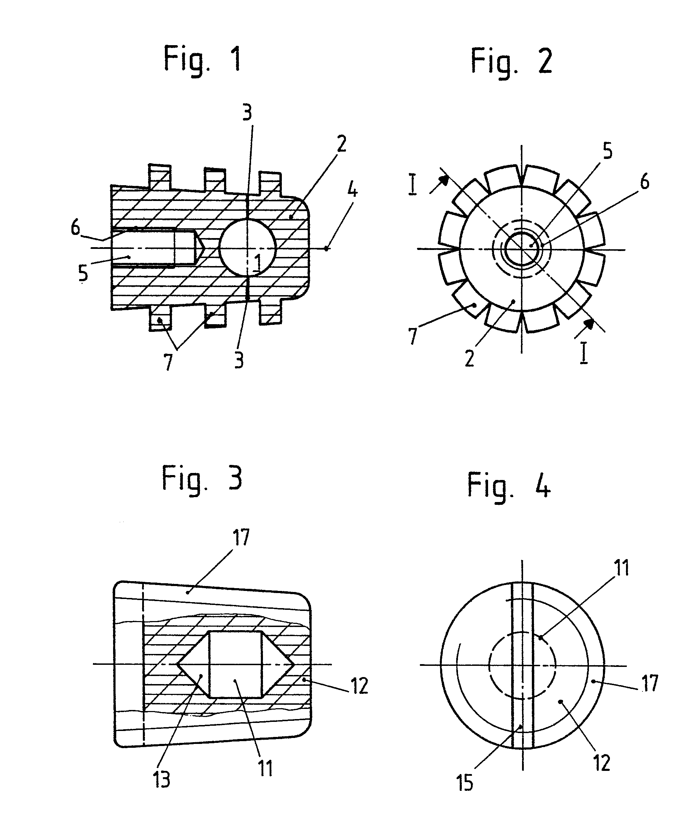

Der Kontrastkörper 1 ist beim ersten Ausführungsbeispiel
nach Fig. 1 und 2 eine Metallkugel, beispielsweise eine
Stahlkugel, von einigen Zehntel Millimetern Durchmesser.
Diese ist in ein flüssigkeitsdichtes Gehäuse als Verankerungskörper 2 eingebettet; der Verankerungskörper 2, der beispielsweise aus Polyäthylen besteht,ist primär
längs der Naht 3 senkrecht zu seiner Längsachse 4 geteilt,
wobei in seinen beiden Teilen halbkugelförmige auf die
Abmessungen des Kontrastkörpers 1 abgestimmte Hohlräume
vorhanden sind. Nach Einlegen des Kontrastkörpers 1 sind
beide Teile des Verankerungskörpers 2 flüssigkeitsdicht
miteinander verschweisst worden.

In die linke Stirnfläche des Verankerungskörpers 2 nach
Fig. 1 ist eine mit der Längsachse koaxiale Bohrung 5
eingebracht, die ein Gewinde 6 enthält; in diesem kann
ein Setz- und/oder, falls erforderlich, ein Ausziehinstrument
befestigt werden.

Auf seinem leicht konisch verlaufenden Mantel trägt der
Verankerungskörper 2 mehrere Kränze von elastischen
lappenartigen Lamellen 7; mit diesen verankert sich der
Verankerungskörper 2 in der Wand von am Knochen angebrachten
Bohrungen in einer solchen Weise, dass er - und damit der
Kontrastkörper 1 - über lange Zeiträume ortsfest im Knochen
fixiert bleibt.

Der Kontrastkörper 11 der zweiten Ausführungsform (Fig. 3 und
4) besteht aus einem mit als Kegelspitzen 13 auslaufenden
Enden versehenen Kreiszylinder, der in einem als Kegelstumpf mit abgerundeten Ecken ausgebildeten Verankerungskörper 12 gelagert, z.B. in diesen, aus Kunststoff bestehende
Körper eingegossen,ist.

Als Struktur für eine Fixierung im Knochen ist in den
konischen Mantel des Verankerungskörpers 12 ein Gewinde 17
eingeschnitten. Die linke Stirnseite des Verankerungskörpers
12 ist mit einem Schlitz 15 versehen, in den ein Einschraubinstrument eingesetzt werden kann.

Während die Kontrastkörper 1 und 11 beispielsweise Abmessungen von Zehntel mm haben, beträgt der Durchmesser der Verankerungskörper 2 und 12 mit Vorteil einige mm, so dass seine Herstellung und das Setzen der für seine Aufnahme vorgesehenen Knochenbohrungen keine Schwierigkeiten bereiten.

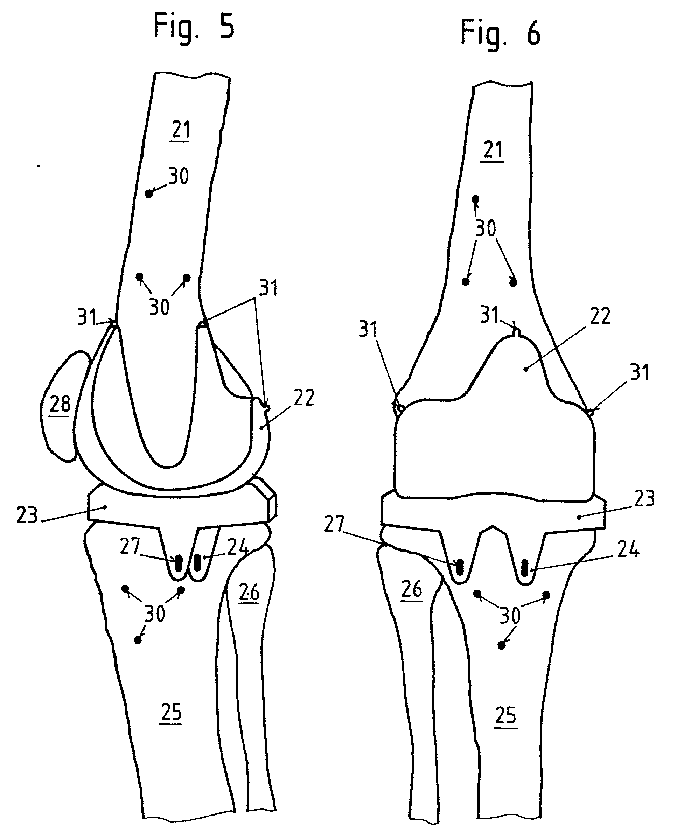

Das in Fig. 5 und 6 gezeigte Kniegelenk ist mit einer Prothese versehen, die aus einem kappenartig die Kondylen des Femurs 21 umschliessenden Femurteil 22 und einem Tibiateil 23 besteht. Der Femurteil 22 ist dabei aus einem der in der Implantat-Technik verwendeten Metalle oder aus einer Metall-Legierung hergestellt, während der Tibiateil 23, der mit Zapfen 24 in der Tibia 25 bzw. dem Wadenbein 26 verankert ist, aus Kunststoff gefertigt ist. Im Plateau des Tibiateils 23 bzw. seinen Zapfen 24 sind kleine Metallstäbchen eingelagert, die eine Identifizierung der Lage des Tibiateils 23 auf einem Röntgenbild erlauben.

Mit 28 ist in Fig. 5 die Patella bezeichnet, die in Fig. 6 nicht sichtbar ist.

Zur Festlegung von ortsfesten Bezugspunkten im Knochen sind im Femur 21 sowie in der Tibia 25 bzw. im Wadenbein 26 erfindungsgemässe Implantate 30 gesetzt, wobei im Ober- und im Unterschenkel drei Elemente 30 in einem Dreieck angeordnet sind. Unter Umständen ist es möglich, für die Aufnahme der Implantate 30 dabei bereits vorhandene, als Hilfsbohrungen während der Implantation gebrauchte Bohrungen zu verwenden.

Ausmessungen der Dreiecksseiten und -winkel ermöglichen bei einem Vergleich zweier Röntgenaufnahmen, Aenderungen des Abbildungsmasstabes und der räumlichen Lage der Knochen zu erkennen und festzustellen. Abstands- und Winkelbestimmungen von den einzelnen Implantaten 30 zu markanten Punkten 31 des Femurteils oder beispielsweise zu den Stäbchen 27 im Tibiateil lassen - unter Berücksichtigung der Massstabs- und Lageverschiedenheiten zweier Aufnahmen - Rückschlüsse auf die Relativbewegung der Prothesenteile gegenüber dem Knochen zu.

**Patentansprüche**

1. Markierungsimplantat zur Festlegung eines Bezugspunktes im menschlichen Knochengewebe, gekennzeichnet durch einen im Röntgenbild zu seiner Umgebung in Kontrast stehenden Kontrastkörper (1, 11), dessen Raumform so ausgebildet ist, dass seine Projektion auf die Filmebene für jede mögliche Einstrahlrichtung eine geometrische Bestimmung desselben Raumpunktes ermöglicht, und ferner durch einen röntgenographisch durchlässigen Verankerungskörper (2, 12), durch den der Kontrastkörper (1, 11) im Knochen ortsfest gehalten ist.

2. Implantat nach Anspruch 1, dadurch gekennzeichnet, dass der Kontrastkörper (1, 11) eine Metallkugel ist.

3. Implantat nach Anspruch 1, dadurch gekennzeichnet, dass der Kontrastkörper (1, 11) fixiert in ein als Verankerungskörper (2, 12) dienendes, flüssigkeitsdichtes Gehäuse eingelegt ist.

4. Implantat nach Anspruch 1, dadurch gekennzeichnet, dass der Verankerungskörper (2, 12) eine zur ortsfesten Fixierung im Knochen geeignete Struktur (7, 17) aufweist.

5. Implantat nach Anspruch 4, dadurch gekennzeichnet, dass der Verankerungskörper (2, 12) Mittel 5, 6; 15) für den Eingriff eines Setz- und/oder Ausziehinstrumentes aufweist.

Fig. 1

Fig. 2

Fig. 3

Fig. 4

Fig. 5

Fig. 6

Europäisches Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | IEEE ENGINEERING IN MEDICINE AND BIOLOGY, Band 2, Nr. 1, März 1983, Seite 5, New York, US; "System predicts success of implants" * Insgesamt * | 1-5 | A 61 B 19/00 A 61 B 6/12 |
| | --- | | |
| A | US-A-4 349 498 (W.H. ELLIS u.a.) * Insgesamt * | 1-4 | |
| | --- | | |
| A | DE-A-2 512 983 (P.L. SAMIS) * Seite 2, Zeilen 22-30; Seite 3, Zeile 12 - Seite 4, Zeile 24; Seite 9, Zeilen 1-12 * | 1-4 | |
| | --- | | |
| A | US-A-4 373 532 (B.C. HILL u.a.) * Spalte 3, Zeilen 4-22; Zusammenfassung * | 2 | RECHERCHIERTE SACHGEBIETE (Int. Cl.4) |
| | --- | | |
| A | US-A-4 202 349 (J.W. JONES) * Spalte 3, Zeilen 30-57 * | 3 | A 61 B A 61 F A 61 C |
| | --- | | |
| A | US-A-3 836 776 (E.H. GULLEKSON) | | |
| | --- | | |
| A | US-A-4 277 389 (H.A. SCHEETZ) | | |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 28-02-1985 | WOLF C.H.S. |

Europäisches
Patentamt

**EUROPÄISCHER RECHERCHENBERICHT**

**0146699**

Nummer der Anmeldung

EP 84 11 1617

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 123 806 (H.C. AMSTUTZ u.a.) * Spalte 6, Zeilen 12-17; Figuren 2,6 * | 1,2 | |
| A | US-A-3 194 239 (C.J.P. SULLIVAN) | 1,2 | |
| A | ENGINEERING IN MEDICINE, Band 10, Nr. 2, April 1981, Seiten 97-105, Whitstable, Kent, GB; D. ELAD u.a.: "Synthesis of a knee joint endoprosthesis is based on pure rolling" * Seite 99, Figur 5 * | 1,2 | |

RECHERCHIERTE
SACHGEBIETE (Int. Cl.4)

Der vorliegende Recherchenbericht wurde für alle Patentansprüche erstellt.

| Recherchenort DEN HAAG | Abschlußdatum der Recherche 28-02-1985 | Prüfer WOLF C.H.S. |
|---|---|---|